# NEUE EUROPÄISCHE PATENTSCHRIFT

(11) **EP 2 296 722 B2**
(45) Veröffentlichungstag und Bekanntmachung des Hinweises auf die Entscheidung über den Einspruch: **12.01.2022**
(45) Hinweis auf die Patenterteilung: 24.08.2016
(21) Anmeldenummer: 09754169.2
(22) Anmeldetag: 16.03.2009
(51) Int. Cl.: A61L 31/16, A61L 29/16

(54) **MEDIZINISCHE EINRICHTUNG UND VERFAHREN ZU IHRER HERSTELLUNG**
MEDICAL DEVICE AND METHOD FOR THE MANUFACTURE THEREOF
SYSTÈME MÉDICAL ET PROCÉDÉ DE PRODUCTION ASSOCIÉ

(30) Priorität: 31.05.2008 DE 202008007346 U; 26.02.2009 DE 102009010401
(43) Veröffentlichungstag der Anmeldung: 23.03.2011
(73) Patentinhaber: Sellin, Lothar, 52074 Aachen (DE); EuroCor GmbH, 53117 Bonn (DE)
(72) Erfinder: SELLIN, Lothar, 52074 Aachen (DE)
(74) Vertreter: Castell, Klaus
(86) Internationale Anmeldenummer: PCT/IB2009/000531
(87) Internationale Veröffentlichungsnummer: WO 2009/144541

(56) Entgegenhaltungen:
- EP-A1- 1 916 006
- WO-A2-2008/063576
- DE-A1-102007 010 354
- US-A1- 2006 099 236
- US-A1- 2007 269 486
- Arteriosclerosis, Trombosis, and Vascular Biology 2005, 25:748-753, 27.01.2005

## Beschreibung

Die Erfindung betrifft eine medizinische Einrichtung mit einem Grundkörper mit einer Beschichtung und ein Verfahren zu ihrer Herstellung.

Medizinische Einrichtungen mit unterschiedlichen Beschichtungen sind aus dem Stand der Technik bekannt. Sie finden im human- und veterinärmedizinischen Bereich insbesondere aber im kardiologischen Bereich Anwendung.

Auch Beschichtungen mit Terpenoiden sind aus dem Stand der Technik zahlreich bekannt.

Terpenoide haben die Eigenschaft gut auf Oberflächen zu haften, abriebfest und mit anderen Materialien gut mischbar zu sein. Terpenoide haben deshalb neben rein technischen Anwendungen ein weites Anwendungsgebiet als Coatingmaterial für oral applizierte Medikamente, Nahrungsergänzungsstoffe sowie im kosmetischen Bereich gefunden. Im kosmetischen Bereich insbesondere im dermatologischen Bereich sind zahlreiche Anwendungen von Schellack publiziert.

In JP 300539737 wird eine Methode zur Herstellung einer Oberflächenbeschichtung von Pulver, Granulaten oder Tabletten, die aktive Ingredienzien, insbesondere selektive Proteine enthalten, beschrieben, um eine Freisetzung erst im Duodenum und nicht im Magen zu erzielen. Bei den verwendeten Materialien werden neben den üblichen synthetischen Materialien z.B. Zein und Schellack genannt.

Eine Kombination von Schellack mit Carbonaten für orale Applikationen wird in JP 10218795 allgemein für den Bereich der (Gesundheits-) Lebensmittel und Medizin ausgeführt.

In JP 6203620 wird ebenfalls Schellack zum Coating von hämoglobinhaltigen Grundstoffen benutzt, um über diese orale Applikation den Eisenmangel bei Anämie zu behandeln.

Die Verwendung von Schellack in der Wundheilung bei Diabetesand Wundbrandbasierten Wunden, Schuppenflechte und ähnlichen Hauterkrankungen wird in EP 167 90 78 angeführt.

Ausgewählte natürlich vorkommende Harze wie Kiefernöl, Kampfer und Kolophonium, deren Bestandteile der Klasse der Terpenoide zugehörig sind, werden in CN 126199 als Rohmaterialien zur Herstellung von multifunktionellen, schnell wirkenden medizinischen Pflastern verwendet. Dabei wird insbesondere die feuchtigkeitsvertreibende, schmerzlindernde, entzündungshemmende und abschwellende Wirkung beschrieben.

Im Patent EP 1666019 wird zu Beschichtungen von natürlichen Zähnen mit Schellack und Kolophonium zum Zwecke der Verbesserung des Aussehens und zum Schutz vor Karies ausgeführt. Weiterhin werden dem Zahnlack verschiedene Pigmente als Verschönerungseffekt zugesetzt.

In WO 03034944 werden Stents mit einer hämokompatiblen Schicht überzogen, die eine oder mehrere weitere Schichten besitzen, wobei diese wiederum mindestens einen antiproliferativen oder/und entzündungshemmenden und bei Bedarf auch antithrombotischen Wirkstoff enthalten. Hier werden als Wirkstoffe u.v.a. auch Terpenoide, die z.B. in der Krebstherapie eingesetzt werden, genannt.

Die Patentanmeldung DE 107 34 544 A1 beschreibt den Zusatz von Schellackdispersionen zu O/W- oder W/O-Emulsionen oder Hydrogelen, um das Hautgefühl bei herkömmlichen Emulsionen und Gelen (Vermeidung eines öligen und oder klebrigen Eindrucks) bei gleichzeitiger Erhöhung der Wasserfestigkeit und zur Erzielung stabiler Emulsionen zu verbessern. Im Bereich der Lebensmittelzusatzstoffe wird Schellack, unter der Bezeichnung E 904 geführt, umfangreich als Coatingmaterial eingesetzt.

Aufgabe der hier vorliegenden Erfindung ist es, verbesserte medizinische Einrichtungen zur Verfügung zu stellen.

Dieses Ziel wird mit einem Ballonkatheter mit einem Grundkörper und einer Beschichtung erreicht, wobei der Ballonkatheter die Merkmale des Patentanspruchs aufweist. Dabei werden die physikalischen sowie antibakteriellen Eigenschaften ebenso ausgenutzt wie die typischen Freisetzungseigenschaften. Die vorliegende Beschichtung mit film- bzw. lackbildenden Terpenoiden gewährleistet sowohl den Schutz der medizinischen Einrichtung, eröffnet aber auch die Möglichkeit, funktionell wirksam zu werden. Dieser funktionelle Aspekt betrifft Materialeigenschaften wie Festigkeit und Elastizität der Beschichtung und die von der Beschichtung ausgehenden Wirkungen, wie z.B. antibakterielle Effekte.

Die medizinische Einrichtung ist ein Ballonkatheter, insbesondere ein PTA- oder PCTA-Katheter. Ballone können alle Ballone sein, die im medizinischen Bereich Anwendung finden und in der Lage sind auf irgendeiner Weise Medikamente zur Verhinderung oder Beseitigung einer Restenose zu applizieren, das heißt abzugeben.

Die in der Beschichtung enthaltenen terpenoiden Materialen können natürlichen, synthetischen oder semisynthetischen Ursprungs sein.

Als besonders vorteilhaft hat sich erwiesen, wenn die Terpenoide einer der folgenden Stoffklassen angehören oder Mischungen daraus enthalten: Monoterpene, Sesquiterpene, Diterpene, Sesterterpene, Triterpene und Tetraterpene.

Zudem kann die Beschichtung weitere Materialien aufweisen, die organischen oder anorganischen Unsprungs sind.

Insbesondere vorteilhaft ist es, wenn die anorganischen Materialien Oxide und/oder Salze sind. Mögliche Salze können Phospate, Sulfate, Silicate, Carbonate oder Mischungen daraus sein. Der Zusatz dieser anorganischen Materialien, spielt eine besondere Rolle für das Einwachsen der Implantate und soll durch die Bereitstellung von entsprechenden Ionen eine Wachstumsstimulation herbeiführen.

In Bezug auf die organischen Materialien haben sich Lipide oder deren Komponenten als vorteilhaft erwiesen. Diese können Carbonsäuren deren Derivate oder Substitutionsprodukte sein. Der Zusatz dieser organischen Materialien bewirkt eine positive Veränderung in der Elastizität der Beschichtung. Die hergestellte Ballonbeschichtung wird druckelastischer und damit weniger spröde.

Es hat sich herausgestellt, dass insbesondere die Terpenoide Schellolsäure und Abietinsäure für sich oder in ihrer natürlich vorkommenden Form als Schellack und Kolophonium über die gewünschten physikalischchemischen Eigenschaften als Beschichtungsmaterial für Ballonkatheter verfügen.

Dem Beschichtungsmaterial wird ein anwendungsspezifisches Additiv zugesetzt. Die anwendungsspezifischen Additive ermöglichen eine breite pharmakologische Wirkung der Beschichtung. Hierbei führt die Beschichtung mit film- bzw. lackbildenden Terpenoiden zu einer Beschleunigung der Wirkstoffpenetration beispielsweise durch die Gefäßwand, wodurch eine verbesserte Bioverfügbarkeit im Gewebe erreicht wird. Dies hat eine erhöhte therapeutische Wirksamkeit zur Folge.

Das Additiv ist ein Medikament zur Reduzierung oder Verhinderung einer Restenose.

Die Zugabe des Additivs kann in Form von soliden Nano- oder Mikropartikeln bzw. -kapseln erfolgen. Neben der direkten Zugabe von bioaktiven Substanzen wie beispielsweise Antibiotika, Zytostatika, Hormonen oder Wachstumsfaktoren bzw. einer Kombination dieser Substanzklasen können die bioaktiven Substanzen auch immobilisiert zum Einsatz kommen. Über die Art und Weise der Immobilisierung/Verkapselung kann eine direkte Freisetzung der Wirksubstanzen über den Ballonkatheter erzielt werden.

Ein weiterer Aspekt der Erfindung betrifft ein Verfahren zum Herstellen einer medizinischen Einrichtung. Die verfahrenstechnische Umsetzung der Herstellung der Beschichtung beginnt in einem vorgelagerten Schritt damit, dass die terpenoide Komponente in eine homogene Lösung überführt wird. Bevorzugte Lösungsmittel können hierbei niedere Alkohole, Ketone und Ester sein. Insbesondere Ethanol kann verwendet werden. Nach Einstellung der gewünschten Konzentration werden das anwendungsspezifische Additiv und gegebenenfalls die anorganischen und/oder organischen Materialien zugesetzt.

Sind alle Komponenten zusammengegeben, erfolgt in Abhängigkeit von der Viskosität der Masse eine Homogenisierung durch verschiedene Rührtechniken (Rührwerke, Dispergatoren). In einem nächsten Schritt wird die Beschichtungslösung durch Tauchen oder Besprühen auf die Oberfläche der medizinischen Einrichtung aufgebracht und in einem nachgelagerten Schritt getrocknet.

Weiterhin kann nicht erfindungsgemäß auch durch Druckminderung das Beschichtungsmaterial auf die Oberfläche des Ballonkatheters aufgebracht werden, z.B. durch Bedampfen in einer Unterdruckkammer.

Dabei ist die Anwendung des Verfahrens nicht nur auf statische Oberflächen beschränkt. Vielmehr kann das Beschichtungsmaterial auf die Oberfläche aufgetragen werden, während die medizinische Einrichtung rotiert.

Im Folgenden werden zwei Vergleichsbeispiele beschrieben.

Nach einem ersten Vergleichsbeispiel werden 5 g Schellack 24 h unter Rühren in 100 ml Ethanol gelöst. Dieser Lösung wird 2 % Stearin (w/w) zugesetzt. Man lässt die Lösung noch weitere 4 h bei 30 °C rühren. In diese Lösung werden die Ballonkatheter getaucht. Durch mehrmaliges Tauchen wird die gewünschte Schichtdicke erreicht. Anschließend wird bei 40 °C 1 h getrocknet.

Nach einem zweiten Vergleichsbeispiel werden 5 g Schellack 24 h unter Rühren in 100 ml Ethanol gelöst. Die Lösung wird mittels Sprühtechnik auf den Ballonkatheter aufgebracht. Es wird anschließend im Tauch oder Sprühverfahren ein Zytostatikum auf der Suspension aufgetragen. Das Zytostatikum wird nach wenigen Minuten (1 - 2 min) abgegeben.

## Patentansprüche

1. Ballonkatheter mit einem Grundkörper mit einer Beschichtung, ***dadurch gekennzeichnet, dass*** die Beschichtung als terpenoide Komponente Schellack und als anwendungsspezifisches Additiv ein Medikament zur Reduzierung oder Verhinderung einer Restenose aufweist, erhältlich durch ein Verfahren mit folgenden Schritten:
(A) Überführen der terpenoiden Komponente mit einem Lösungsmittel in eine homogene Lösung,
(B) Einstellen einer Konzentration,
(C) Zusammensetzen des anwendungsspezifischen Additivs,
(D) Homogenisieren der Masse aus Lösungsmittel, terpenoider Komponente und anwendungsspezifischem Additiv in Abhängigkeit der Viskosität durch Rührtechniken und
(E) Aufbringen der Beschichtungslösung auf die Oberfläche des Ballonkatheters durch Tauchen oder Besprühen.

## Claims

1. Balloon catheter having a base body with a coating, ***characterized in that*** the coating comprises shellac as terpenoid component and a medicament for reducing or preventing restenosis as application-specific additive, obtainable by a process comprising the following steps:
(A) converting the terpenoid component with a solvent into a homogeneous solution,
(B) adjusting a concentration,
(C) compounding the application-specific additive,
(D) homogenizing the mass of solvent, terpenoid component and application-specific additive as a function of viscosity by stirring techniques, and
(E) applying the coating solution to the surface of the balloon catheter by dipping or spraying.

## Revendications

1. Cathéter à ballonnet ayant un corps de base avec un revêtement, ***caractérisé en ce que*** le revêtement comprend de la gomme-laque comme composant terpénoïde et un médicament pour réduire ou empêcher la resténose comme additif spécifique à l'application, pouvant être obtenu par un procédé comprenant les étapes suivantes :
(A) la transformation du composant terpénoïde avec un solvant en une solution homogène,
(B) l'ajustement d'une concentration,
(C) l'assemblage de l'additif spécifique à l'application,
(D) homogénéiser la masse de solvant, de composant terpénoïde et d'additif spécifique à l'application en fonction de la viscosité par des techniques d'agitation, et
(E) appliquer la solution de revêtement à la surface du cathéter à ballonnet par trempage ou pulvérisation.
